(19) Europäisches Patentamt / European Patent Office / Office européen des brevets

(11) **EP 4 510 014 A1**

(12) **EUROPEAN PATENT APPLICATION**

(43) Date of publication:
19.02.2025   Bulletin 2025/08

(21) Application number: 24187385.0

(22) Date of filing: **09.07.2024**

(51) International Patent Classification (IPC):
G06F 17/16 (2006.01)          G01J 1/00 (2006.01)
G06F 17/00 (2019.01)         G06F 18/2135 (2023.01)
G06F 18/22 (2023.01)          G01J 3/28 (2006.01)

(52) Cooperative Patent Classification (CPC):
G06F 17/16; G06F 18/2135; G06F 18/22;
G06V 10/7715

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: **15.08.2023   JP 2023132273**

(71) Applicant: **FUJITSU LIMITED**
**Kawasaki-shi, Kanagawa 211-8588 (JP)**

(72) Inventor: **KATAOKA, Yuji**
**Kawasaki-shi, Kanagawa, 211-8588 (JP)**

(74) Representative: **Hoffmann Eitle**
**Patent- und Rechtsanwälte PartmbB**
**Arabellastraße 30**
**81925 München (DE)**

(54)   **ARITHMETIC PROGRAM, ARITHMETIC METHOD, AND INFORMATION PROCESSING DEVICE**

(57)   An arithmetic program comprising instructions which, when executed by a computer, cause the computer to execute processing including: generating a first vector by extracting each of elements of a matrix of a plurality of pieces of analysis data represented as two-dimensional data; creating a first normalized vector by normalizing each of the elements of the first vector; generating a second vector by extracting each of elements of a matrix of characteristic data that corresponds to the plurality of pieces of analysis data; creating a second normalized vector by normalizing each of the elements of the second vector; and specifying a correspondence relationship between the element included in the first vector and the element included in the second vector according to similarity between the first normalized vector and the second normalized vector.

## FIG. 6A

**Description**

FIELD

The present case is related to an arithmetic program, an arithmetic method, and an information processing device.

BACKGROUND

A technique for analyzing analysis data obtained by analyzing a substance to be analyzed has been disclosed.
Japanese Laid-open Patent Publication No. 2005-010931, Japanese Laid-open Patent Publication No. 2007-164772, and Japanese Laid-open Patent Publication No. 2018-119897 are disclosed as related art.

SUMMARY

TECHNICAL PROBLEM

There has been a need to predict a material composition, a substance structure, a chemical state, and the like that exhibit desired physical properties and functions by associating the obtained analysis data with characteristic data including physical properties and the like. In view of the above, a technique utilizing mathematical and statistical science has been proposed. The mathematical and statistical science technique mainly used is regression analysis. However, in the regression analysis, results to be obtained include inaccurate results despite a complicated processing process of selecting an appropriate hyperparameter $\lambda$.
In one aspect, an object of the present case it to provide an arithmetic program, an arithmetic method, and an information processing device capable of obtaining a highly accurate result.

SOLUTION TO PROBLEM

According to an aspect of the embodiments, there is provided a non-transitory computer-readable recording medium storing an arithmetic program for causing a computer to execute processing including: generating a first vector by extracting each of elements of a matrix of a plurality of pieces of analysis data represented as two-dimensional data; creating a first normalized vector by normalizing each of the elements of the first vector; generating a second vector by extracting each of elements of a matrix of characteristic data that corresponds to the plurality of pieces of analysis data; creating a second normalized vector by normalizing each of the elements of the second vector; and specifying a correspondence relationship between the element included in the first vector and the element included in the second vector according to similarity between the first normalized vector and the second normalized vector.

ADVANTAGEOUS EFFECTS OF INVENTION

A highly accurate result may be obtained.

BRIEF DESCRIPTION OF DRAWINGS

FIG. 1 is a diagram exemplifying an X-ray diffraction spectrum;
FIG. 2 is a diagram exemplifying set objective variables and explanatory variables;
FIG. 3 is a diagram exemplifying an analysis result;
FIG. 4 is a diagram exemplifying a result of a change in a regression coefficient;
FIG. 5 is a diagram for examining whether or not the result illustrated in FIG. 4 is correct;
FIG. 6A is a functional block diagram exemplifying an overall configuration of an information processing device, and
FIG. 6B is a block diagram exemplifying a hardware configuration of the information processing device;
FIG. 7 is a flowchart illustrating exemplary operation of the information processing device;
FIG. 8 is a flowchart illustrating exemplary operation of the information processing device;
FIG. 9 is a flowchart illustrating exemplary operation of the information processing device;
FIG. 10 is a diagram exemplifying a calculation result; and
FIG. 11 is a diagram exemplifying the calculation result.

DESCRIPTION OF EMBODIMENTS

In order to develop new materials and new substances having new physical properties and functions or to improve physical properties and functions of existing materials and existing substances, physical analysis and chemical analysis of the materials and the substances are performed. In this physical and chemical analysis, a mass-to-charge ratio (m/z) in a case of mass analysis, a wave number (1/cm) in a case of infrared spectroscopic analysis, and a diffraction angle ($2\theta$) in a case of X-ray diffraction analysis are used as parameters. Analysis data obtained by the analysis is obtained as two-dimensional data, such as a spectrum in which signal intensity (detection signal value) on the vertical axis obtained by each detector is recorded for a changed parameter with the horizontal axis as a parameter. Alternatively, in a case of electronic microscope observation, it is obtained as two-dimensional data such as an image in which gradation obtained by an accompanying detector is recorded as a detection signal value for a changed parameter with a scanning position of an electron beam as a parameter.

Those pieces of analysis data are associated with characteristic data including physical properties and the like to predict a material composition, a substance structure, a chemical state, and the like that exhibit desired physical properties and functions. In this association, it is highly important to find out which part of the two-dimensional data is closely related to the characteristic. For example, the association is carried out by repeating construction and verification of a temporary structure based on the physical principle and the chemical principle, which needs a high degree of expertise and wide experience, and in addition, it often takes a lot of time.

In view of the above, a technique utilizing mathematical and statistical science has been proposed for the purpose of improving efficiency. The mathematical and statistical science technique mainly used is regression analysis. In the regression analysis, a characteristic value is used as an objective variable, and two-dimensional data is used as an explanatory variable. Then, the explanatory variable having the largest regression coefficient obtained as a result of the regression analysis is considered as a part most closely related to the objective variable. In recent years, in order to suppress overtraining, the regression analysis using regularization has been utilized to improve analysis accuracy. The regression analysis using regularization is a technique as follows.

As in the following equation (1), it is assumed that there are m + 1 explanatory variables y for n + 1 objective variables s. Both the objective variable s and the explanatory variable y are represented by vectors.

[Math. 1]

$$\boldsymbol{s} = (s_0, \ s_1, \ \cdots, \ s_n), \quad \boldsymbol{y} = (y_0, \ y_1, \ \cdots, \ y_m) \quad \cdots \cdots \cdots (1)$$

Each of a prediction value and a regression coefficient is expressed as the following equation (2). Both the prediction value and the regression coefficient are represented by vectors.

[Math. 2]

$$\widehat{\boldsymbol{y}} = (\widehat{y}_0, \ \widehat{y}_1, \ \cdots, \ \widehat{y}_m), \quad \boldsymbol{w} = (w_0, \ w_1, \ \cdots, \ w_n) \quad \cdots \cdots \cdots (2)$$

In this case, a linear regression equation may be expressed by the following equation (3). Note that $w_0 y_0$ represents an intercept.

[Math. 3]

$$\widehat{y} = w_0 y_0 + w_1 y_1 + w_2 y_2 + \cdots + w_n y_m \quad \cdots \cdots \cdots (3)$$

The following equation (4) is obtained when the equation (3) described above is expressed by a vector.

[Math. 4]

$$\widehat{\boldsymbol{y}} = (y_0, \ y_1, \ y_2, \cdots, \ y_m) \begin{pmatrix} w_0 \\ w_1 \\ \vdots \\ w_m \end{pmatrix} \quad \cdots \cdots \cdots (4)$$

In multiple regression analysis, the number of the prediction values described above is not one, and the regression coefficient is derived from a large number of pieces of training data. Thus, the number of prediction values is equal to the number of pieces of training data. A sample size is n + 1, which is the number of objective variables, and is expressed as the following equation (5).

[Math. 5]

$$\begin{pmatrix} \hat{y}_0 \\ \hat{y}_1 \\ \hat{y}_2 \\ \hat{y}_3 \\ \hat{y}_4 \\ \hat{y}_5 \\ \vdots \\ \hat{y}_n \end{pmatrix} = \begin{pmatrix} y_{00} & y_{10} & \cdots & y_{m0} \\ y_{01} & y_{11} & \cdots & y_{m1} \\ y_{02} & y_{12} & \cdots & y_{m2} \\ y_{03} & y_{13} & \cdots & y_{m3} \\ y_{04} & y_{14} & \cdots & y_{m4} \\ y_{05} & y_{15} & \cdots & y_{m5} \\ \vdots & \vdots & \ddots & \vdots \\ y_{0n} & y_{1n} & \cdots & y_{mn} \end{pmatrix} \begin{pmatrix} w_0 \\ w_1 \\ w_2 \\ w_3 \\ w_4 \\ w_5 \\ \vdots \\ w_n \end{pmatrix} \qquad \cdots \cdots \cdots \cdots (5)$$

A weight w (regression coefficient) is specified such that there is no isolation between the prediction value and the actual s, which is the objective variable. In the multiple regression analysis, a least squares method is used to determine a parameter such that a loss function L expressed by the following equation (6) is minimized.

[Math. 6]

$$L = \sum_{i=0}^{n} (s_i - \hat{y}_i)^2 \qquad \cdots \cdots \cdots \cdots \cdots \cdots \cdots \cdots (6)$$

In the regularization, a function obtained by adding a regularization term to the loss function L is set as a new minimization target. Examples of a representative regularization term include an L2 regularization term of the following equation (7) and an L1 regularization term of the following equation (8). Note that $\lambda$ represents a hyperparameter that may be freely determined.

[Math. 7] L2 regularization:

$$R(w) = \lambda \sum_{i=0}^{n} w_j^2 \qquad \cdots \cdots \cdots \cdots (7)$$

[Math. 8] L1 regularization:

$$R(w) = \lambda \sum_{j=0}^{n} |w_j| \qquad \cdots \cdots \cdots (8)$$

When the loss function to which the regularization term is added is minimized, a coefficient of a variable that does not contribute to the objective variable is made smaller and the substantial number of explanatory variables is reduced as compared with the case of not adding the regularization term, and an effect of suppressing overtraining may be obtained. By manipulating $\lambda$ of the regularization term, it becomes possible to manipulate the influence of the regularization. As the value of $\lambda$ increases, the influence of the regularization term increases, and the number of items having a coefficient value of 0 increases in an explanatory variable group. Specifically, it results in an optimization problem of a regression function as expressed in the following equation (9), and the regression coefficient w is optimized (L1 regularization) as in the following equation (10).

[Math. 9]

$$S_\lambda(w) = \sum_{i=0}^{m} \{s_i - (w_0 y_{i0} + w_1 y_{i1} + \cdots + w_n y_{in})\}^2 + \lambda \sum_{j=0}^{n} |w_j| \qquad \cdots \cdots \cdots \cdots (9)$$

[Math. 10]

$$\widehat{\boldsymbol{w}} = argmin_w \left[ \sum_{i=0}^{m} \{s_i - (w_0 y_{i0} + w_1 y_{i1} + \cdots + w_n y_{in})\}^2 + \lambda \sum_{j=1}^{n} |\boldsymbol{w}_j| \right] \quad \cdots (10)$$

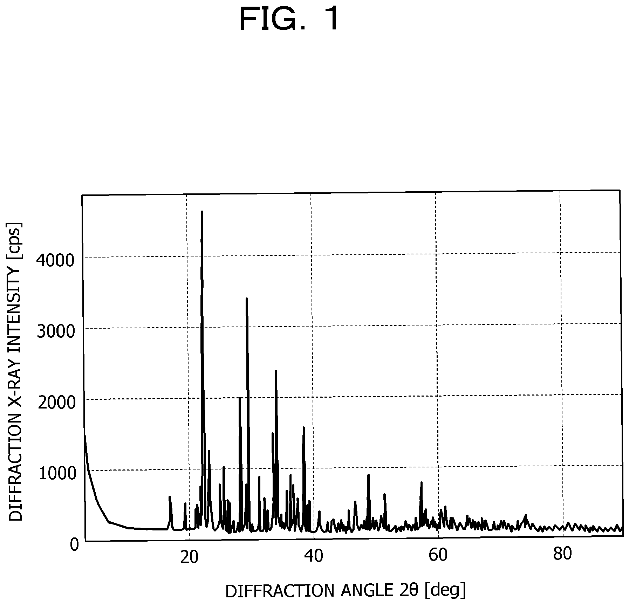

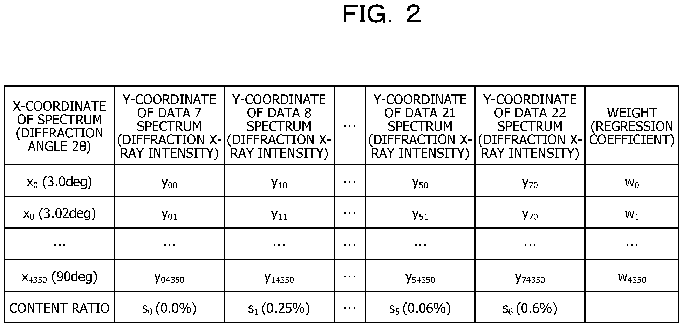

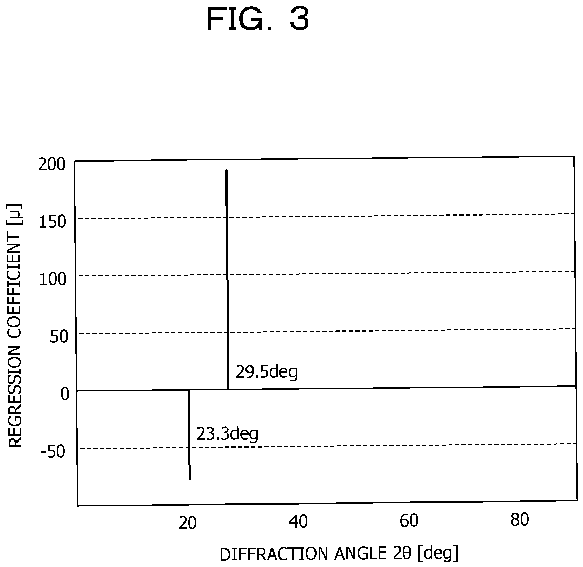

An example of the regression analysis using the regularization will be described below. For example, it is assumed that an X-ray diffraction spectrum of a certain material is obtained as illustrated in FIG. 1. It is assumed that the X-ray diffraction spectrum in FIG. 1 is represented as a sum of eight spectra having different ratios of a certain substance contained. Thus, the eight spectra are included as components in individual intensity values of the X-ray diffraction spectrum in FIG. 1. When regularized regression analysis is carried out by comparing the eight spectra (Data 7, Data 8, Data 9, Data 10, Data 17, Data 19, Data 21, and Data 22) and characteristics corresponding thereto (abundance ratio of certain element: 0%, 0.25%, 0.5%, 0.75%, 0.11%, 0.14%, 0.06%, and 0.46%) with the equations (5) to (8) described above and setting the objective variables and the explanatory variables as in FIG. 2, the result of FIG. 3 is obtained.

FIG. 3 illustrates that only two diffraction angles 23.3 deg and 29.5 deg of a group of 4,350 explanatory variables (diffraction angles) have regression coefficients, and regression coefficients related to other diffraction angles are 0. However, 23.3 deg indicates a negative value, and 29.5 deg indicates a positive value. A negative value indicates that, in the X-ray diffraction spectrum, the intensity at 23.3 deg and the characteristic (abundance ratio of certain element) are in inverse proportion, and a positive value indicates a proportional relationship.

As described above, while an easy-to-understand result is obtained in FIG. 3, the hyperparameter $\lambda$ needs to be set to an appropriate value in the process of regularization. As a method of setting $\lambda$, a technique of scanning the value of $\lambda$ within a certain range is adopted. As described above, as the value of $\lambda$ increases, the number of items having the coefficient value of 0 increases in the explanatory variable group, and the regression coefficients are gradually narrowed down.

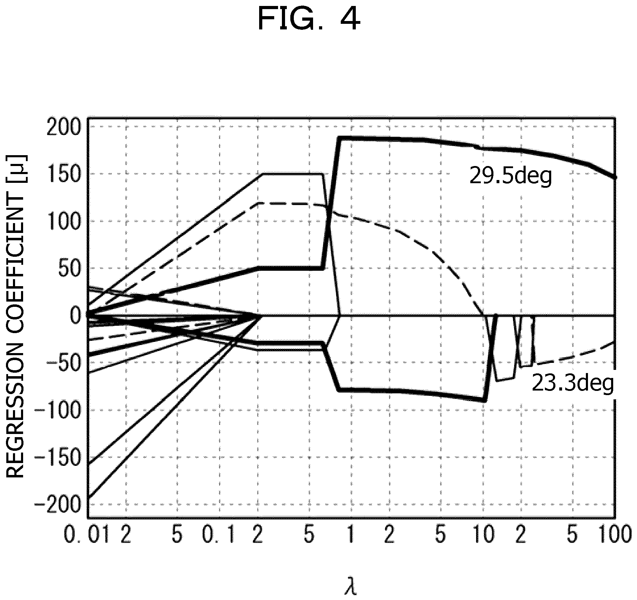

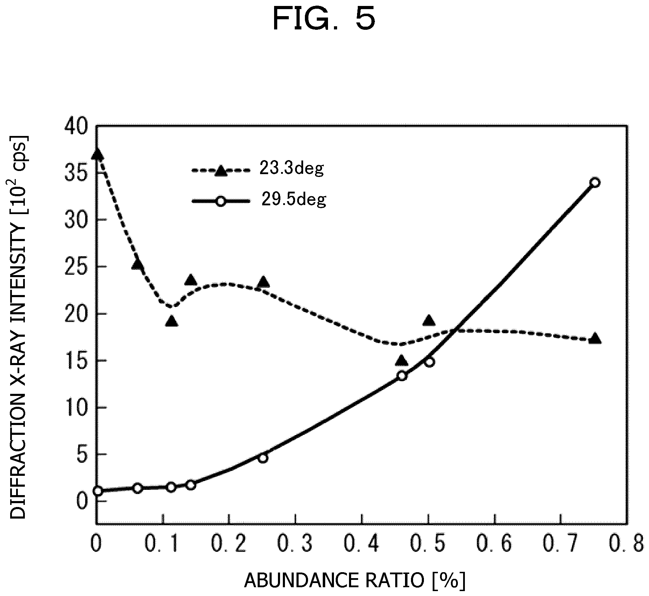

Then, an appropriate item is selected from the narrowed regression coefficients, and the value of the explanatory variable related thereto is set as a target value, that is, a part exhibiting the closest relationship with the characteristic in the spectrum. For example, when $\lambda$ is scanned from 0.01 to 100 and the change in the regression coefficient associated therewith is examined, the result of FIG. 4 is obtained. When the value of $\lambda$ increases, two of 23.3 deg and 29.5 deg are finally left. FIG. 3 is a diagram obtained by assuming that the regression coefficient remaining when $\lambda = 50$ is an appropriate value according to FIG. 3. FIG. 5 is a diagram for examining whether or not the result illustrated in FIG. 4 is correct. The diffraction X-ray intensity at 29.5 deg increases as the abundance ratio increases, and it is found that this part is closely related to the abundance ratio. On the other hand, the diffraction X-ray intensity at 23.3 deg tends to slightly decrease as the abundance ratio increases and is recognized to be related to the abundance ratio to some extent, but a close relationship is hardly recognized.

In view of those results, it may be said that, according to the technique described above, results to be obtained include inaccurate results despite the complicated processing process of selecting an appropriate hyperparameter $\lambda$. Thus, in the following embodiment, an exemplary case where a highly accurate result may be obtained without undergoing a complicated processing process will be described.

[First Embodiment]

First, a principle of a first embodiment will be described.

For the two-dimensional data such as a spectrum, an image, and the like obtained as a result of the physical and chemical analysis, when m + 1 vertical axis values y corresponding to individual pieces of x with respect to m + 1 horizontal axis values x are obtained as analysis data, each of them is expressed as the following equation (11). The horizontal axis value x and the vertical axis value y are represented by vectors.

[Math. 11]

$$\boldsymbol{x} = (x_0, \ x_1, \ \cdots, \ x_m), \quad \boldsymbol{y} = (y_0, \ y_1, \ \cdots, \ y_m) \quad \cdots \cdots (11)$$

Furthermore, when there are n + 1 pieces of analysis data (e.g., spectra) expressed by the equation (11) described above and there are n + 1 characteristic values s corresponding to the respective pieces of analysis data as in the following equation (12), the analysis data may be expressed as a matrix Y and a characteristic value s as in the following equation (13).

[Math. 12]

$$\boldsymbol{s} = (s_0, \ s_1, \ \cdots, \ s_n) \quad \cdots \cdots \cdots (12)$$

[Math. 13]

$$Y = \begin{pmatrix} y_{00} & y_{10} & \cdots & y_{m0} \\ y_{01} & y_{11} & \cdots & y_{m1} \\ y_{02} & y_{12} & \cdots & y_{m2} \\ y_{03} & y_{13} & \cdots & y_{m3} \\ y_{04} & y_{14} & \cdots & y_{m4} \\ y_{05} & y_{15} & \cdots & y_{m5} \\ \vdots & \vdots & \ddots & \vdots \\ y_{0n} & y_{1n} & \cdots & y_{mn} \end{pmatrix}, \quad s = \begin{pmatrix} s_0 \\ s_1 \\ s_2 \\ s_3 \\ s_4 \\ s_5 \\ \vdots \\ s_n \end{pmatrix} \quad \cdots \cdots (13)$$

Here, each column of Y is expressed as the following equation (14) to be extracted as a first vector. Note that the vector of the equation (12) described above is a second vector.

[Math. 14]

$$y_i = (y_{i0}, \ y_{i1}, \ \cdots, \ y_{in}), \quad i = 0, \ 1, \ \cdots, m \quad \cdot \cdot (14)$$

Next, $y_i$ is normalized to create a first normalized vector, and s is normalized to create a second normalized vector. For example, the normalization is carried out with an $L^p$ norm, which is a length of each vector expressed by the following equations (15) and (16). As a result, the normalization is carried out such that the sum of the detection signal values becomes constant.

[Math. 15]

$$L_s^p = \|s\|_p = \sqrt[p]{|s_0|^p + |s_1|^p + \cdots |s_n|^p} \quad \cdots \cdots \cdots (15)$$

[Math. 16]

$$L_{yi}^p = \|y_i\|_p = \sqrt[p]{|y_{i0}|^p + |y_{i1}|^p + \cdots |y_{in}|^p} \quad (i = 0, 1, \cdots m) \quad \cdots \cdot (16)$$

The normalized value is expressed by the following equations (17) and (18).

[Math. 17]

$$z_s = \frac{s}{\|s\|_p} \quad \cdots \cdots \cdots \cdots \cdots \cdots (17)$$

[Math. 18]

$$z_{yi} = \frac{y_i}{\|y_i\|_p} \quad \cdots \cdots \cdots \cdots \cdots \cdots (18)$$

Next, similarity between $z_s$ and $z_{yi}$ is calculated. For example, as the similarity between $z_s$ and $z_{yi}$, cosine similarity is obtained as in the following equation (19). Next, $z_{yi}$ closest to 1 and $z_{yi}$ closest to -1 in the following equation (19) are extracted.

[Math. 19]

$$\cos(x, y) = \frac{\langle z_s, z_{yi} \rangle}{\|z_s\| \|z_{yi}\|} \quad \cdots \cdots \cdots \cdots (19)$$

As the cosine similarity is closer to 1, the similarity is larger, and the direction of the vector is closer to the opposite direction as the cosine similarity is closer to -1. Thus, $z_{yi}$ having the cosine similarity closest to 1 has the strongest positive relationship with $z_s$, and $z_{yi}$ having the cosine similarity closest to -1 has the strongest negative relationship. Here, positive and negative mean proportional and inversely proportional, respectively. It may be said that $x_i$ giving this $z_{yi}$ is a portion where the strongest relationship with the characteristic is exhibited in the two-dimensional data.

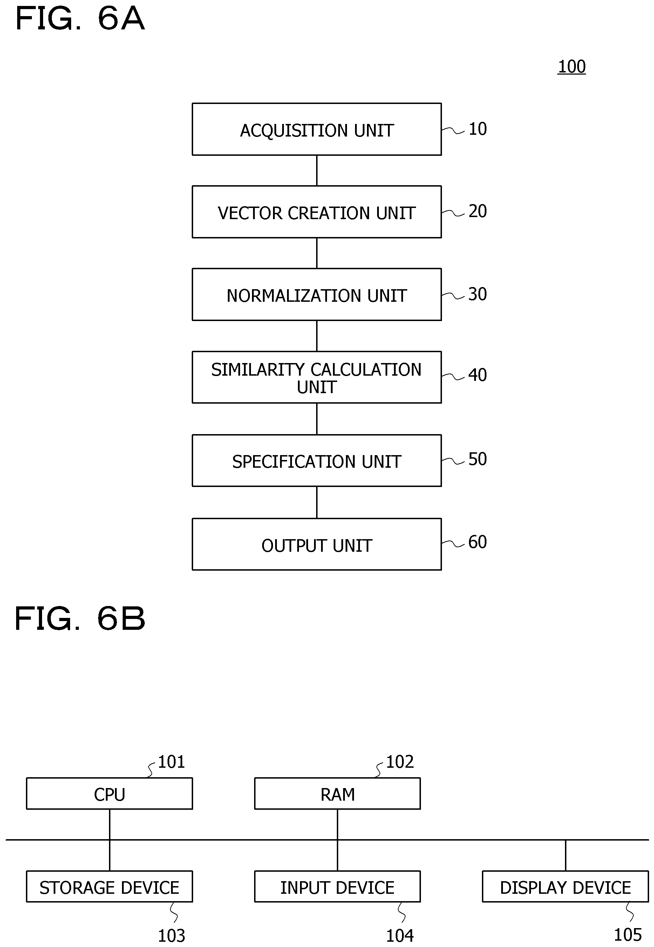

Next, a specific device configuration and the like of the present embodiment will be described. FIG. 6A is a functional block diagram exemplifying an overall configuration of an information processing device 100 according to the first embodiment. As exemplified in FIG. 6A, the information processing device 100 functions as an acquisition unit 10, a vector creation unit 20, a normalization unit 30, a similarity calculation unit 40, a specification unit 50, an output unit 60, and the like.

FIG. 6B is a block diagram exemplifying a hardware configuration of the information processing device 100. As exemplified in FIG. 6B, the information processing device 100 includes a central processing unit (CPU) 101, a random access memory (RAM) 102, a storage device 103, an input device 104, a display device 105, and the like.

The CPU 101 is a central processing device. The CPU 101 includes one or more cores. The RAM 102 is a volatile memory that temporarily stores a program to be executed by the CPU 101, data to be processed by the CPU 101, and the like. The storage device 103 is a nonvolatile storage device. As the storage device 103, for example, a read only memory (ROM), a solid state drive (SSD) such as a flash memory, a hard disk to be driven by a hard disk drive, or the like may be used. The storage device 103 stores an arithmetic program. The input device 104 is an input device such as a keyboard or a mouse. The display device 105 is a display device such as a liquid crystal display (LCD). The CPU 101 executes the arithmetic program to implement the acquisition unit 10, the vector creation unit 20, the normalization unit 30, he similarity calculation unit 40, the specification unit 50, the output unit 60, and the like. Note that hardware such as a dedicated circuit may be used as the acquisition unit 10, the vector creation unit 20, the normalization unit 30, the similarity calculation unit 40, the specification unit 50, the output unit 60, and the like.

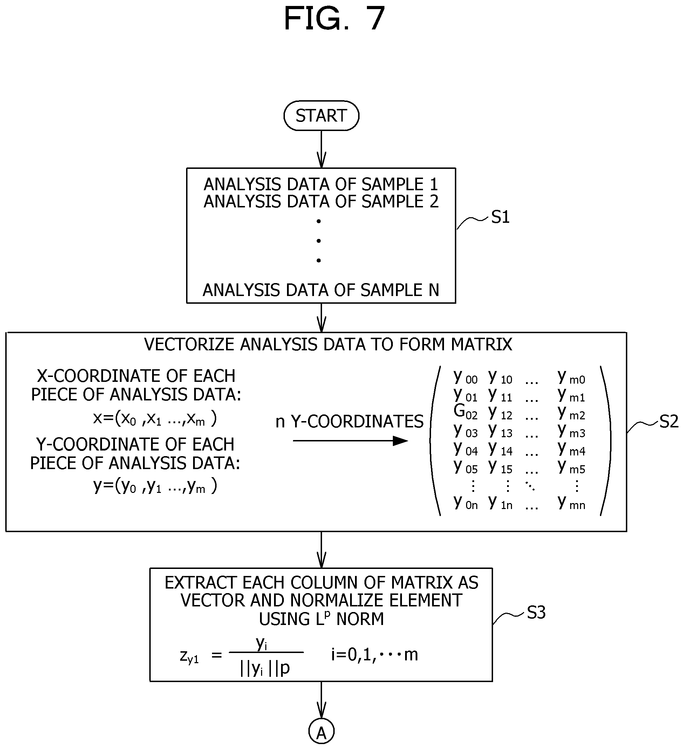

FIGs. 7 to 9 are flowcharts illustrating exemplary operation of the information processing device 100. As exemplified in FIG. 7, the acquisition unit 10 obtains analysis data of samples 1 to n (step S1). The analysis data is two-dimensional data, which is the two-dimensional data in which signal intensity (detection signal value) on the vertical axis obtained by a detector is recorded for a changed parameter with the horizontal axis as a parameter.

Next, the vector creation unit 20 expresses each piece of the analysis data obtained in step S1 with a vector as in the equation (13) described above (step S2). However, it is assumed that there are m + 1 y-coordinates for m + 1 x-coordinates for each piece of the analysis data.

Next, the normalization unit 30 extracts each column of the matrix obtained in step S2 as a vector, and normalizes the element as in the equation (18) described above using the $L^p$ norm (step S3).

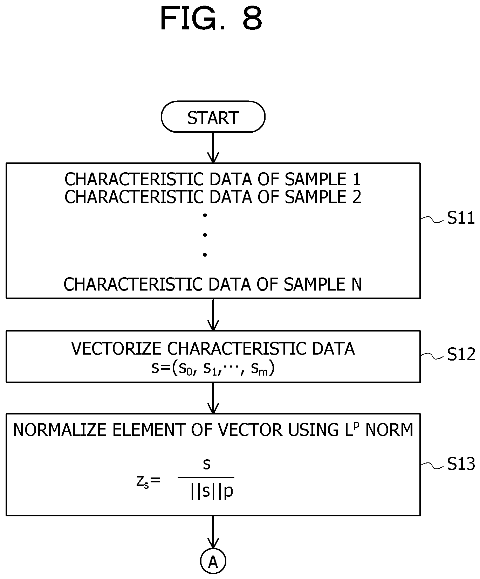

In parallel with steps S1 to S3, as exemplified in FIG. 8, the acquisition unit 10 obtains characteristic data of the samples 1 to n (step S11).

Next, the vector creation unit 20 expresses each piece of the characteristic data obtained in step S11 with a vector as in the equation (13) described above (step S12).

Next, the normalization unit 30 normalizes the element of the matrix obtained in step S12 as in the equation (17) described above using the $L^p$ norm (step S13).

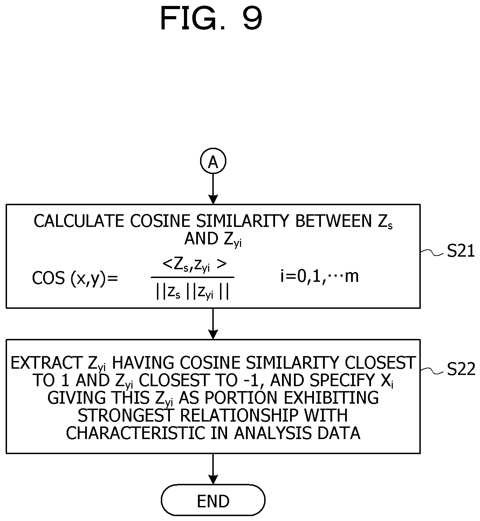

After the execution of step S3 and after the execution of step S13, as exemplified in FIG. 9, the similarity calculation unit 40 calculates cosine similarity $\cos(x, y)$ between $z_s$ and $z_{yi}$ as in the equation (19) described above (step S21).

Next, the specification unit 50 extracts $z_{yi}$ having the cosine similarity closest to 1 and $z_{yi}$ closest to -1, and specifies $x_i$ giving this $z_{yi}$ as a portion that exhibits the strongest relationship with the characteristic in the analysis data (step S22). The output unit 60 outputs a result specified by the specification unit 50. The output result is displayed by the display device 105, for example. For example, the display device 105 graphically displays the relationship between the characteristic value and the detection signal value (y-coordinate value) in the extracted parameter value (x-coordinate value).

As described above, according to the present embodiment, each of elements of the matrix of multiple pieces of analysis data represented as two-dimensional data is extracted as the first vector, and the first normalized vector normalized for each of the elements is obtained. Furthermore, each of elements of the matrix of the characteristic data corresponding to the multiple pieces of analysis data is extracted as the second vector, and the second normalized vector normalized for each of the elements is obtained. A correspondence relationship between the element included in the first vector and the element included in the second vector is obtained according to the similarity between the first normalized vector and the second normalized vector. In this manner, it becomes possible to obtain a highly accurate result without undergoing the complicated processing process of selecting an appropriate hyperparameter.

Next, the usefulness of the present embodiment will be described with respect to the eight X-ray diffraction spectra (Data 7, Data 8, Data 9, Data 10, Data 17, Data 19, Data 21, and Data 22) described above. The characteristic value s represents an abundance ratio of a certain element, and $y_i$ represents a y-coordinate of each of the eight spectra having 4,351 x-coordinates. The characteristic value s, the x-coordinate, and yi may be expressed by the following equations (20) to (22).

[Math. 20]

$$s = (0, 0.25, 0.5, 0.75, 0.11, 0.14, 0.06, 0.46) \cdots \cdots (20)$$

[Math. 21]

$$x = (x_0, x_1, \cdots, y_{4350}) \cdots \cdots \cdots \cdots \cdots (21)$$

[Math. 22]

$$y_i = (y_{i0}, y_{i1}, \cdots, y_{i7}), \quad i = 0, 1, \cdots, 4350 \cdots \cdots (22)$$

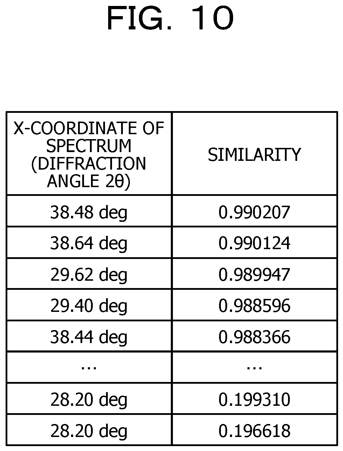

The cosine similarity was calculated for the equations (20) and (22) described above based on the equations (14) to (19) described above to obtain the result of FIG. 10. Note that an $L^1$ norm of p = 1 was used as a norm.

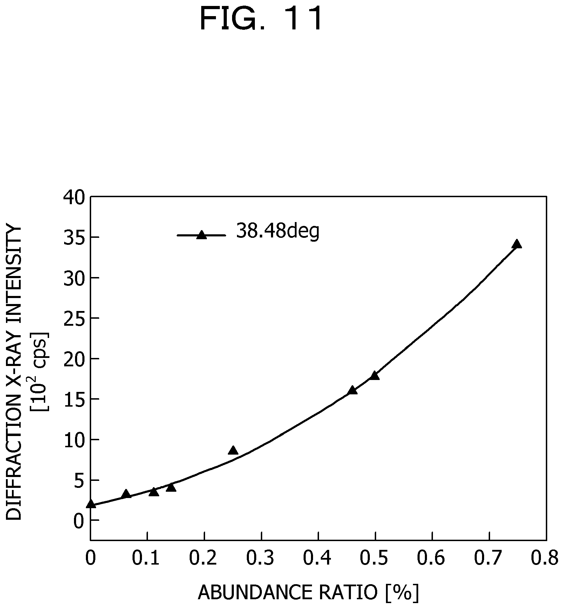

In FIG. 10, the diffraction X-ray intensity at the diffraction angle of 38.48 deg with the similarity closest to 1 is plotted against the abundance ratio to obtain FIG. 11. As illustrated in FIG. 11, a favorable proportional relationship is obtained. Note that, as may be seen from FIG. 10, there is no result indicating a negative value of the similarity in the results according to the present embodiment. Therefore, in the spectrum, no inverse proportion is exhibited with the abundance ratio.

While FIGs. 10 and 11 illustrate the results using the $L^1$ norm, similar results may be obtained using an $L^2$ norm, $L^3$ norm, or $L^4$ norm. Furthermore, the result of FIG. 11 may also be utilized as a highly accurate calibration curve in searching analysis data for unknown characteristics.

As described above, according to the present embodiment, the similarity evaluation is performed with the cosine similarity after the normalization with the $L^p$ norm, whereby a portion that exhibits a strongest relationship with the characteristic in the two-dimensional data, such as a spectrum, an image, and the like, may be extracted. FIG. 11 indicates that the result is appropriate. Furthermore, while complicated work is involved to search for an appropriate hyperparameter, a favorable result may be obtained by simple calculation according to the present embodiment. Moreover, the accuracy is clearly represented by a numerical value of the similarity. On the other hand, while information regarding a negative relationship is extracted according to the technique using regularized regression analysis, FIG. 4 indicates that the information is inaccurate. As described above, it may be said that the present embodiment is a more useful technique.

While the embodiment has been described above in detail, the embodiment is not limited to such specific embodiment, and various modifications and alterations may be made within the scope of the gist of the embodiment described in the claims.

## Claims

1. An arithmetic program comprising instructions which, when executed by a computer, cause the computer to execute processing comprising:

   generating a first vector by extracting each of elements of a matrix of a plurality of pieces of analysis data represented as two-dimensional data;
   creating a first normalized vector by normalizing each of the elements of the first vector;
   generating a second vector by extracting each of elements of a matrix of characteristic data that corresponds to the plurality of pieces of analysis data;
   creating a second normalized vector by normalizing each of the elements of the second vector; and
   specifying a correspondence relationship between the element included in the first vector and the element included in the second vector according to similarity between the first normalized vector and the second normalized vector.

2. The non-transitory computer-readable recording medium according to claim 1, wherein when the first vector and the second vector are normalized, a norm is used for the normalization.

3. The non-transitory computer-readable recording medium according to claim 1, wherein

   as the matrix of the plurality of pieces of analysis data, a vertical axis coordinate is extracted when a parameter is set to a horizontal axis in the plurality of pieces of analysis data,
   cosine similarity is calculated as the similarity between the first normalized vector and the second normalized vector, and

a vector with the cosine similarity closest to 1 and a vector with the cosine similarity closest to -1 are extracted from the first normalized vector, and a coordinate that gives each of the extracted vectors is specified from a horizontal axis coordinate of the plurality of pieces of analysis data.

4. The non-transitory computer-readable recording medium according to claim 1, wherein the analysis data includes spectrum data or image data.

5. An arithmetic method implemented by a computer, the arithmetic method comprising:

generating a first vector by extracting each of elements of a matrix of a plurality of pieces of analysis data represented as two-dimensional data;
creating a first normalized vector by normalizing each of the elements of the first vector;
generating a second vector by extracting each of elements of a matrix of characteristic data that corresponds to the plurality of pieces of analysis data;
creating a second normalized vector by normalizing each of the elements of the second vector; and
specifying a correspondence relationship between the element included in the first vector and the element included in the second vector according to similarity between the first normalized vector and the second normalized vector.

6. An information processing apparatus comprising:

a vector creation unit configured to generate a first vector by extracting each of elements of a matrix of a plurality of pieces of analysis data represented as two-dimensional data, and generate a second vector by extracting each of elements of a matrix of characteristic data that corresponds to the plurality of pieces of analysis data;
a normalization unit configured to create a first normalized vector by normalizing each of the elements of the first vector, and create a second normalized vector by normalizing each of the elements of the second vector; and
a specification unit configured to specify a correspondence relationship between the element included in the first vector and the element included in the second vector according to similarity between the first normalized vector and the second normalized vector.

# FIG. 1

# FIG. 2

| X-COORDINATE OF SPECTRUM (DIFFRACTION ANGLE 2θ) | Y-COORDINATE OF DATA 7 SPECTRUM (DIFFRACTION X-RAY INTENSITY) | Y-COORDINATE OF DATA 8 SPECTRUM (DIFFRACTION X-RAY INTENSITY) | ... | Y-COORDINATE OF DATA 21 SPECTRUM (DIFFRACTION X-RAY INTENSITY) | Y-COORDINATE OF DATA 22 SPECTRUM (DIFFRACTION X-RAY INTENSITY) | WEIGHT (REGRESSION COEFFICIENT) |
|---|---|---|---|---|---|---|
| $x_0$ (3.0deg) | $y_{00}$ | $y_{10}$ | ... | $y_{50}$ | $y_{70}$ | $w_0$ |
| $x_0$ (3.02deg) | $y_{01}$ | $y_{11}$ | ... | $y_{51}$ | $y_{70}$ | $w_1$ |
| ... | ... | ... | ... | ... | ... | ... |
| $x_{4350}$ (90deg) | $y_{04350}$ | $y_{14350}$ | ... | $y_{54350}$ | $y_{74350}$ | $w_{4350}$ |
| CONTENT RATIO | $s_0$ (0.0%) | $s_1$ (0.25%) | ... | $s_5$ (0.06%) | $s_6$ (0.6%) | |

# FIG. 3

# FIG. 4

# FIG. 5

## FIG. 6A

100

| ACQUISITION UNIT | ~10 |
|---|---|

| VECTOR CREATION UNIT | ~20 |
|---|---|

| NORMALIZATION UNIT | ~30 |
|---|---|

| SIMILARITY CALCULATION UNIT | ~40 |
|---|---|

| SPECIFICATION UNIT | ~50 |
|---|---|

| OUTPUT UNIT | ~60 |
|---|---|

## FIG. 6B

101

| CPU |
|---|

102

| RAM |
|---|

| STORAGE DEVICE |
|---|

103

| INPUT DEVICE |
|---|

104

| DISPLAY DEVICE |
|---|

105

# FIG. 7

START

ANALYSIS DATA OF SAMPLE 1
ANALYSIS DATA OF SAMPLE 2
.
.
.
ANALYSIS DATA OF SAMPLE N

~S1

VECTORIZE ANALYSIS DATA TO FORM MATRIX

X-COORDINATE OF EACH
PIECE OF ANALYSIS DATA:

$$x=(x_0, x_1 ..., x_m)$$

Y-COORDINATE OF EACH
PIECE OF ANALYSIS DATA:

$$y=(y_0, y_1 ..., y_m)$$

n Y-COORDINATES $\longrightarrow$

$$\begin{pmatrix} y_{00} & y_{10} & ... & y_{m0} \\ y_{01} & y_{11} & ... & y_{m1} \\ G_{02} & y_{12} & ... & y_{m2} \\ y_{03} & y_{13} & ... & y_{m3} \\ y_{04} & y_{14} & ... & y_{m4} \\ y_{05} & y_{15} & ... & y_{m5} \\ \vdots & \vdots & \ddots & \vdots \\ y_{0n} & y_{1n} & ... & y_{mn} \end{pmatrix}$$

~S2

EXTRACT EACH COLUMN OF MATRIX AS
VECTOR AND NORMALIZE ELEMENT
USING $L^p$ NORM

$$z_{y1} = \frac{y_i}{||y_i||p} \quad i=0,1,\cdots m$$

~S3

A

# FIG. 8

START

CHARACTERISTIC DATA OF SAMPLE 1
CHARACTERISTIC DATA OF SAMPLE 2
.
.
.
CHARACTERISTIC DATA OF SAMPLE N
$\sim$ S11

VECTORIZE CHARACTERISTIC DATA
$s = (s_0, s_1, \cdots, s_m)$
$\sim$ S12

NORMALIZE ELEMENT OF VECTOR USING $L^p$ NORM

$$z_s = \frac{s}{||s||p}$$
$\sim$ S13

Ⓐ

# FIG. 9

Ⓐ

↓

CALCULATE COSINE SIMILARITY BETWEEN $Z_s$ AND $Z_{yi}$

$$COS(x,y) = \frac{<Z_s, Z_{yi}>}{||Z_s|| ||Z_{yi}||} \qquad i = 0, 1, \cdots m$$

～S21

↓

EXTRACT $Z_{yi}$ HAVING COSINE SIMILARITY CLOSEST TO 1 AND $Z_{yi}$ CLOSEST TO -1, AND SPECIFY $X_i$ GIVING THIS $Z_{yi}$ AS PORTION EXHIBITING STRONGEST RELATIONSHIP WITH CHARACTERISTIC IN ANALYSIS DATA

～S22

↓

END

# FIG. 10

| X-COORDINATE OF SPECTRUM (DIFFRACTION ANGLE 2θ) | SIMILARITY |
|---|---|
| 38.48 deg | 0.990207 |
| 38.64 deg | 0.990124 |
| 29.62 deg | 0.989947 |
| 29.40 deg | 0.988596 |
| 38.44 deg | 0.988366 |
| ... | ... |
| 28.20 deg | 0.199310 |
| 28.20 deg | 0.196618 |

# FIG. 11

Europäisches Patentamt

European Patent Office

Office européen des brevets

# EUROPEAN SEARCH REPORT

**Application Number**

EP 24 18 7385

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 114 529 741 A (FLYING BOOK DEEP NOO DIGITAL SCIENCE AND TECH SHANGHAI STOCK LIMITED C) 24 May 2022 (2022-05-24) * the whole document * | 1-6 | INV. G06F17/16 G01J1/00 G06F17/00 G06F18/2135 |
| A | EP 3 166 128 B1 (THERMO FINNIGAN LLC [US]) 27 June 2018 (2018-06-27) * paragraphs [0010], [0016], [0020], [0023], [0024], [0026], [0119], [0122], [0126], [0127], [0158] * | 1-6 | G06F18/22  ADD. G01J3/28 |
| A | CN 105 224 961 A (41ST INST CHINA ELECTRONICS TECHNOLOGY GROUP CORP) 6 January 2016 (2016-01-06) * the whole document * | 1-6 | |

**TECHNICAL FIELDS SEARCHED (IPC)**

G06F
G01J

The present search report has been drawn up for all claims

| Place of search | Date of completion of the search | Examiner |
|---|---|---|
| Munich | 18 November 2024 | Kleppmann, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 7385

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2024

| Patent document cited in search report | | Publication date | Patent family member(s) | | Publication date |
|---|---|---|---|---|---|
| CN 114529741 | A | 24-05-2022 | NONE | | |
| EP 3166128 | B1 | 27-06-2018 | EP 3166128 A1 | | 10-05-2017 |
| | | | US 2017133215 A1 | | 11-05-2017 |
| CN 105224961 | A | 06-01-2016 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005010931 A
- JP 2007164772 A

- JP 2018119897 A